(19) Europäisches Patentamt
European Patent Office
Office européen des brevets

(11) Veröffentlichungsnummer: **0 455 101 B1**

(12) **EUROPÄISCHE PATENTSCHRIFT**

(45) Veröffentlichungstag der Patentschrift: **05.07.95**

(51) Int. Cl.[6]: **C12P 19/44**, C07K 9/00, C07H 15/04

(21) Anmeldenummer: **91106494.7**

(22) Anmeldetag: **23.04.91**

(54) **Verfahren zur glycosidasekatalysierten Synthese von Glycokonjugaten.**

(30) Priorität: **25.04.90 DE 4013077**

(43) Veröffentlichungstag der Anmeldung:
**06.11.91 Patentblatt 91/45**

(45) Bekanntmachung des Hinweises auf die Patenterteilung:
**05.07.95 Patentblatt 95/27**

(84) Benannte Vertragsstaaten:
**BE CH DE FR GB IT LI NL SE**

(56) Entgegenhaltungen:
**EP-A- 0 226 563**

**TRENDS IN BIOTECHNOLOGY. Bd. 6, Nr. 10, Okfober 1988, CAMBRIDGE GB Seiten 256 - 264; KURT G.I. NILSSON: 'Enzymatic synthesis of oligosaccharides'**

(73) Patentinhaber: **HOECHST AKTIENGESELLSCHAFT**

**D-65926 Frankfurt (DE)**

(72) Erfinder: **Holla, Wolfgang, Dr.**
**Am Obertor 30**
**W-6238 Hofheim/Taunus (DE)**
Erfinder: **Schudok, Manfred, Dr.**
**Birminghamstrasse 89**
**W-6230 Frankfurt am Main (DE)**

**EP 0 455 101 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Glycokonjugate (Glycoproteine, Glycosphingolipide, Glycophospholipide) spielen eine zentrale Rolle bei biologischen Erkennungsprozessen, wie der Tumorgenese, der bakteriellen und viralen Infektion, der Zell-Zell-Erkennung, dem Zellwachstum und der Zelldifferenzierung. Sie bilden die Grundlage der Blutgruppenklassifizierung und sind verantwortlich für die Internalisierung verschiedener makromolekularer Stoffe und Medikamente. Ein wichtiges Einsatzgebiet von Glycoproteinen ist daher z.B. die selektive Adressierung von Pharmaka an das Zielorgan sowie der Schutz von Medikamenten vor proteolytischem Abbau (H. Schachter, Clinical Biochemistry 17, (1984) 3; N. Sharon, Trends Biochem. Sci. 9 (1984) 198; S. Hakamori, Cancer Res. 45 (1985) 2405; S. Hakamori, Scientific American 254 (1986) No. 5, 32).

Vor dem Hintergrund der genannten Funktionen von Glycokonjugaten beansprucht deren Synthese besonderes Interesse.

In Glycoproteinen sind Peptid- und Kohlenhydratteil meist kovalent miteinander verbunden. Trotz der Vielfalt der Strukturen ist die Verknüpfungsregion charakteristisch und variiert nur wenig. Es gibt in der Natur hauptsächlich drei Verknüpfungstypen: die N-glycosidische Bindung zwischen N-Acetylglucosamin und der Amidfunktion des Asparagins, die α-O-glycosidische Bindung zwischen N-Acetylgalactosamin und der Hydroxyfunktion von Serin bzw. Threonin und die ß-O-glycosidische Verknüpfung von Xylose oder Galactose an verschiedene Hydroxyaminosäuren.

In einigen interessanten Glycosphingolipiden findet man darüber hinaus ß-O-glycosidische Verknüpfungen von Glucose mit dem serinähnlichen Ceramidteil. Ceramide bestehen aus Sphingosin [(2S, 3R, 4E)-2-Amino-4-octadecen-1,3-diol], Dehydrosphingosin (Sphinganin) oder Phytosphingosin (4-D-Hydroxysphinganin), die mit langkettigen Fettsäuren ($C_{14}$-$C_{26}$) an der 2-Aminogruppe acyliert sind (Y.-T. Li, S.-C. Li, Adv. Carbohydr. Chem. Biochem. 40 (1982) 235).

Zentrale Probleme der chemischen Glycokonjugat-Synthesen sind somit die stereo- und regioselektive Aneinanderreihung von Monosaccharid-Einheiten zu Oligosacchariden, die stereoselektive Knüpfung der glycosidischen Bindung zum Aglycon und nicht zuletzt die Synthese des Aglycons, z.B. eines Peptids oder Ceramids.

Die Lösung dieser Probleme erfordert, auch schon bei kleineren Glycopeptiden, ausgefeilte Synthesekonzepte mit maßgeschneiderten Schutzgruppenkombinationen aus Kohlenhydrat- und Peptidchemie. Chemische Glycoprotein-Synthesen sind daher oft vielstufige, langwierige und häufig nur geringe Mengen der gewünschten Substanz - frei von allen Schutzgruppen - liefernde Reaktionen [H. Kunz, Angew. Chem. 99 - (1987) 297-311); R.R. Schmidt in "Stereochemistry of Organic and Bioorganic Transformations", W. Bartmann und K. B. Sharpless, Hrsg., S. 169, Verlag Chemie Weinheim (1987); H. Paulsen et al. Starch/Stärke 40 (1988) 465-472].

Bei der Synthese von Oligosacchariden finden zunehmend Enzyme aus der Biosynthese dieser Substanzen Anwendung. Die damit verbundenen hohen Stereo- und Regioselektivitäten sowie die Vermeidung aufwendiger Schutzgruppenchemie ermöglichen, im Vergleich zur vielstufigen chemischen Synthesen, häufig gute Ausbeute.

Kürzlich wurden nun erstmals chemoenzymatische Glycoprotein-Synthesen beschrieben [C. Augé, C. Gautheron und H. Pora, Carbohydr. Res. 193 , 288 (1989); J. Thiem und T. Wiemann, Angew. Chem. 102, 78 (1990); C. Unverzagt, H. Kunz und J.C. Paulson, J. Am. Chem. Soc. 112, 9308 (1990)]. Hierbei wurden chemisch synthetisierte Konjugate aus Peptiden bzw. Aminosäuren und einem Zucker enzymatisch am Kohlenhydratteil glycosidiert, d.h. über eine Zucker-Zucker-Verknüpfung verlängert.

Bei den genannten, zur Knüpfung der sacchariden Bindungen verwendeten Enzymen handelt es sich um Glycosyltransferasen. Diese gelten bezüglich des Glycosyldonors und des Acceptors als hochspezifisch und benötigen zur Glycosidierung aktivierte Zucker wie z.B. UDP-Glucose. Die schlechte Zugänglichkeit der Glycosyltransferasen und der aktivierten Zucker läßt jedoch eine breite Anwendung in vitro bzw. Synthesen in größerem Maßstab nicht zu.

Die präparative Synthese von Glycopeptiden (Glycokonjugaten) durch enzymatische Verknüpfung von Mono- oder Oligosacchariden mit einer Aminosäure oder einem Peptid ist bislang weder mit Transferasen noch mit anderen Enzymen beschrieben worden [G.M. Whitesides et al., Tetrahedron 45 (1989), 5365-5422].

Glycosidasen katalysieren in natürlichen Systemen die Hydrolyse glycosidischer Bindungen. Im Laufe der letzten Jahre wurden Glycosidasen in zunehmendem Maße - durch Umkehrung der hydrolytischen Reaktion - auch zur präparativen Synthese von Glycosiden genutzt (R. Wallenfels, R. Weil in "The Enzymes", 3. Ed.; Boyer, P.D. (Ed), Academic Press, New York 1972, Vol. VII, p. 618; J.E.G. Barnett, Int. J. Biochem. 6 (1975) 321-328; G.M. Whitesides et al., Tetrahedron 45 (1989) 5365-5422; Kurt G. Nilsson, Trends in Biotechn. 1988, 256). Die als Nucleophil eingesetzten Hydroxyverbindungen sind einfache

primäre oder sekundäre Alkohole, einige Monosaccharide und in einigen wenigen Fällen auch Steroide. Hauptsächliche Nachteile dieser Synthesen sind die niedrigen Ausbeuten und vielfach die Bildung von Nebenprodukten.

Es wurde nun gefunden, daß D- und L-Serin bzw. D- und L-Serin-Derivate ebenfalls als gute Nucleophile fungieren können. Damit ist man erstmals in der Lage in vitro mit Hilfe eines von der Natur für diese Produktklasse nicht vorgesehenen Enzyms die glycosidische Verknüpfung von Hydroxyaminosäuren bzw. deren Derivaten mit Monosacchariden zu bewerkstelligen. Es konnte somit erstmals gezeigt werden, daß die Synthese von Glycokonjugaten durch direkte Verknüpfung von Zuckern mit D- und L-Serin, D- und L-Serin-Derivaten und D- und L-Serin-Peptiden mit Hilfe von Glycosidasen möglich ist. Dies ist insbesondere überraschend, da Glycosidasen durch N-haltige Monosaccharid-Analoga wie 1-Desoxynojirimycin [N. Peyrieras et al., Embo J. 2, 823 (1983)], bicyclische N-haltige Verbindungen wie Swainsonin [8α,β-Indolizidin-1α,2α,8 β-triol.; R.T. Schwarz, R. Datema, Trends Biochem. Sci. 9, 32 (1984)], aber auch durch einfache N-haltige Alkohole wie Ethanolamin [R. Wallenfels, R. Weil in "The Enzymes", 3. Ed., Boyer, P.D. (Ed); Academic Press, New York 1972, Vol. VII, p. 618] inhibiert werden. Aminosäuren bzw. deren Derivate hätten somit ebenfalls als Inhibitoren wirken können.

Die resultierenden Verbindungen entsprechen zum Teil partiell geschützten, natürlichen Glykokonjugaten bzw. stellen insbesondere Abwandlungen dieser dar und können als Synthese-Vorstufen größerer Konjugate dienen.

Die Erfindung betrifft somit ein Verfahren zur Herstellung von Glycokonjugaten der Formel I,

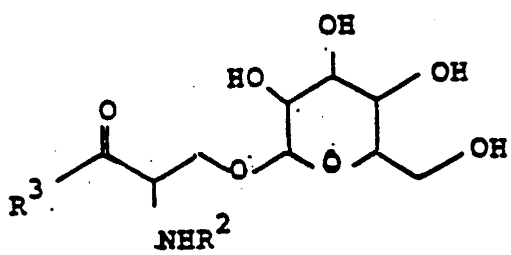

I

in der $R^2$ eine Aminoschutzgruppe und $R^3$ eine Hydroxylgruppe, eine Alkoxy- oder Thioalkylgruppe oder eine Alkenyloxygruppe jeweils mit 1 bis 18 C-Atomen, die mit Halogen oder Cyan substituiert sein können, eine Aryloxygruppe mit 6 bis 10 C-Atomen, die mit Alkyl- Alkoxy-, Thioalkyl-, jeweils mit 1 bis 5 C-Atomen, und Nitrogruppen substituiert sein kann, oder die Gruppe $-NHR^4$ ist, in der $R^4$ eine Alkylgruppe mit 1 bis 5 C-Atomen oder ein Rest der Formel IV

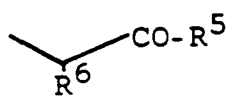

IV

oder ein Di- oder Tripeptid-Rest der Formel V oder VI

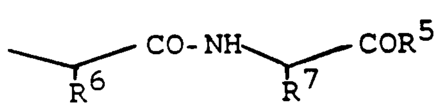

V

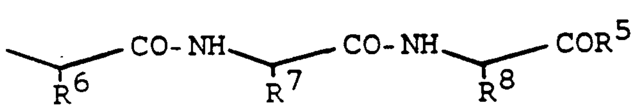

VI

ist, wobei
$R^5$ eine Hydroxylgruppe, eine Alkoxy-, eine Thioalkyl oder eine Alkenyloxygruppe, mit jeweils 1 bis 5 C-Atomen, die mit Halogen oder Cyan substituiert sein kann, eine Aryloxygruppe mit 6 bis 10 C-Atomen, die mit Alkyl-, Alkoxy-, Thioalkyl-, mit jeweils 1 bis 5 C-Atomen, und Nitrogruppen substituiert sein kann und
$R^6$, $R^7$, $R^8$, die gleich oder verschieden sind, Wasserstoff oder geradkettige, verzweigte oder cyclische Alkyl- oder Alkenylgruppen mit 1 bis 10 C-Atomen, die mit Halogen, Hydroxyl, Alkoxy, Thiol, Thioalkyl, Aryl oder Heteroaryl substituiert sein können, darstellen,
das dadurch gekennzeichnet ist, daß die Verbindung der Formel II,

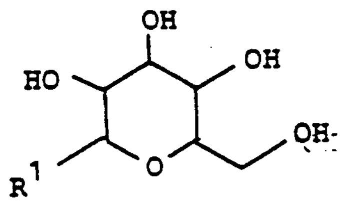

II

in der $R^1$ Fluor, eine Hydroxylgruppe, eine Alkoxygruppe mit 1 bis 5 C-Atomen, eine Alkenyloxygruppe mit 2 bis 5 C-Atomen, eine Aryloxygruppe mit 6 bis 10 C-Atomen oder ein über ein Sauerstoffatom gebundener Kohlenhydratrest ist, mit der Verbindung der Formel III,

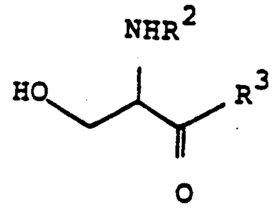

III

in Gegenwart einer Glycosidase inkubiert wird.

Die Verbindung der Formel II kann bevorzugt Glucose, Galactose und Mannose bedeuten. Als bevorzugte Alkoxygruppe in $R^1$-Stellung der Formel II wird eine Methoxygruppe, als bevorzugte Alkenyloxygruppe eine Allyloxy- oder Vinyloxygruppe eingesetzt. Die Aryloxygruppe kann mit elektronenziehenden Substituenten, z.B. Nitro-, Cyanogruppen, Halogen, substituiert sein. Bevorzugt wird eine Phenoxy, eine ortho- oder para-Nitrophenoxy- oder Dinitrophenoxygruppe verwendet. Das durch den Kohlenhydratrest in $R^1$-Stellung gebildete Saccharid ist bevorzugt Maltose oder Lactose. Insbesondere bevorzugt ist $R^1$ als Fluor oder para- bzw. ortho-Nitrophenoxygruppe.

Als Schutzgruppe in $R^2$-Stellung der Formel III können im wesentlichen die in der Peptid- und Glycopeptidchemie gängigen Aminoschutzgruppen eingesetzt werden, wie z.B. Acylgruppen, auch Acylreste längerkettiger Fettsäuren und Alkyl- bzw. Aryloxycarbonylgruppen. Anwendbare Schutzgruppen werden beispielsweise in dem Artikel von H. Hubbuch in Kontakte 3/79, S. 14, in T.W. Greene, Protective Groups in Organic Synthesis, John Wiley & Sons 1981, S. 223 ff. oder in Houben-Weyl Bd 15/1 S. 46 beschrieben. Bevorzugt werden Benzyloxycarbonyl (Z), Allyloxycarbonyl (Aloc) und tertiär-Butyloxycarbonyl (Boc) sowie Trichlorethoxycarbonyl, Formyl, Acetyl, Chloracetyl, Trifluoracetyl, Phenacetyl, Benzoyl oder Acylreste längerkettiger Fettsäuren mit 6 bis 24 C-Atomen eingesetzt.

Der Rest in $R^3$-Stellung sowie $R^5$ aus den Formeln IV-VI sind bevorzugt Methoxy-, Methoxymethyl, Benzyloxymethyl-, Methylthiomethyl, Ethoxy-, Chlorethoxy, Bromethoxy- oder Cyanethoxy- sowie Vinyloxygruppen und gängige Carboxylschutzgruppen der Peptidchemie (Houben-Weyl Bd. 15/1, S. 315 ff.), wie z.B. Benzyloxy-, Trichlorethoxy-, p-Nitrobenzyloxy-, p-Methoxybenzyloxy-, Piperonyloxy-, Allyloxy oder tertiär-Butyloxy- und tertiär-Butyldimethylsilyloxygruppen.

Wenn $R^3$ die Aminogruppe $NHR^4$ bedeutet, ist $R^4$ bevorzugt die Gruppe der Formel IV und V, in der $R^6$ und $R^7$, die gleich oder verschieden sind, Wasserstoff oder geradkettige, verzweigte oder cyclische Alkyl- oder Alkenylgruppen mit 1 bis 10 C-Atomen, die mit Halogen, Hydroxy, Thiol, Alkoxy, Thioalkyl, Aryl oder Heteroaryl substituiert sein können, darstellen. Die Substituenten von $R^6$ und $R^7$ stellen im wesentlichen die Seitenketten neutraler, aliphatischer, aromatischer oder cyclischer $\alpha$-Aminosäuren dar. Insbesondere bevor-

zugt werden als Verbindung der Formel III Z-SerOAllyl, Z-Ser-Ala-OMe, Z-Ser-Leu-OMe, Aloc-Ser-Gly-OEtCl, Boc-Ser-OAllyl und Aloc-Ser-Phe-OMe, verwendet. Wenn $R^2$ in der Verbindung der Formel III einen Acylrest langkettiger Fettsäuren mit 6 bis 24 C-Atomen und $R^3$ eine Alkoxygruppe mit 6 bis 18 C-Atomen bedeutet, so stellt die Verbindung der Formel III eine strukturell Sphingosin- oder Sphinganin-ähnliche Verbindung dar, die als Modellsubstanz für die glycosidasekatalysierten Synthesen von Glycosphingolipiden interessant sind.

Die Substrate der Formel II und III sind entweder käuflich (z.B. Nitrophenylglycoside) oder nach an sich bekannten Methoden leicht herstellbar (Glycosylfluoride nach: F. Micheel, A. Klemer, Adv. Carbohydr. Chem. 16, 85 (1961); DOS 30 40 805; DOS 34 32 565; Aminosäuren und Peptide bzw. geschützte Derivate nach: Houben-Weyl Bände 15/I, 15/II).

Die Verbindungen der Formel II und der Formel III können im Verhältnis 4 zu 1 bis 1 zu 10, bevorzugt 3 zu 2 bis 1 zu 4, eingesetzt werden. Pro mmol Glycosyldonor III verwendet man zweckmäßig 4 bis 40 Units des Enzyms.

Die Reaktion kann in einem pH-Bereich von 5,0 bis 8,0, vorteilhaft jedoch zwischen pH 6,0 und 7,5 ablaufen. Als Puffer sind zu verwenden: HEPES, TRICIN, TAPS, MES, TES, sowie MOPS, CHES, TRIS, K - u. Na-Phosphatpuffer. Die Molarität sollte zwischen 0,01 und 1,0, vorzugsweise zwischen 0,01 und 0,1 liegen. Die Temperatur sollte dabei zwischen ca. -30 °C und 50 °C, bevorzugt zwischen 20 °C und 35 °C, gehalten werden. Oberhalb von 50 °C wird das Enzym mit steigender Temperatur zunehmend irreversibel desaktiviert. Der Inkubationszeitraum kann 1 bis 30 Stunden betragen.

Es werden vorteilhaft Glucosidasen aus Hefe und Süßmandeln, Galactosidasen aus E. coli, Kaffeebohnen, Rindertestes und aus Aspergillus niger, Mannosidasen aus Mandeln, Jack bean und snail aceton powder, Amylasen aus Aspergillus oryzae, Bacillus subtilis und Schweinepankreas, Amyloglucosidasen aus Aspergillus niger sowie eine Transglucosidase verwendet.

Wenn der Glycosyldonor Glucose oder ein 1-Gluco-hexopyranosid ist, wird insbesondere bevorzugt Glucosidase als Enzym verwendet. Wenn jedoch Galactose oder ein 1-Galacto-hexopyranosid bzw. Mannose oder ein 1-Manno-hexopyranosid eingesetzt wird, ist Galactosidase bzw. Mannosidase ein vorteilhaft zu verwendendes Enzym. Während also beim Glycosyldonor II eine verhältnismäßig große Substratspezifität besteht, ist man bei der Wahl der Verbindung der Formel III flexibel. So ist eine Vielzahl unterschiedlicher N- und Carboxyl-Schutzgruppen frei kombinierbar.

Zur Verbesserung der Lösung der Substrate II und III können Lösungsmittel verwendet werden, die das Enzym in seiner Aktivität nicht oder nur geringfügig negativ beeinflussen. Dies sind z.B. Aceton, Dimethoxyethan, Diglyme, insbesondere aber Diisopropyl- u. tert. Butylmethylether, Toluol und Xylol. Ferner können zur Erhöhung der Umsetzungsgeschwindigkeit Salze zugesetzt werden, die mit dem verwendeten Enzymen physiologisch verträglich sind. Solche Salze sind beispielsweise $MnSO_4$, $CaCl_2$, KCl, NaBr, LiCl, LiBr sowie $KMnO_4$, bevorzugt sind jedoch $NiSO_4$ und $MgCl_2$.

Die erfindungsgemäß verwendeten Glycosidasen können als freies wasserlösliches Enzym oder in wasserunlöslicher Form nach herkömmlichen Methoden an einen Träger gebunden (vgl. DOS 27 32 301) in einer wäßrigen Lösung eingesetzt werden. Wenn das Enzym in immobilisierter Form verwendet wird, kann dies sowohl im Batch- als auch im kontinuierlichen Verfahren geschehen.

Der Verlauf der Reaktion kann mit Hilfe des HPLC [ODS-Hypersil™ 5 µm 4,6 x 250 mm, MeOH-$H_2O$, Bioselect™ 100/10-18 250 x 4,6 mm, $CH_3CN$-$H_2O$] oder per DC [$CHCl_3$/Hex/MeOH = 3:1:1] verfolgt werden.

Die anschließende Aufarbeitung erfolgt beispielsweise durch Extraktion mit Toluol oder Diisopropylether, Behandlung mit XAD-Adsorbentien (z.B. XAD-2) zur Nitrophenol-Entfernung, Gefriertrocknung der wäßrigen Phase und Reinigung durch Chromatographie, z.B. präparative Dünnschichtchromatographie, Chromatographie an Kieselgel (Flash- oder konventionell), Flash-Chromatographie oder MPLC an Eurosil Bioselect™ 100-30 C18 (Knauer) oder LiChroPrep™ RP 18 (Merck) sowie Chromatographie an Sephadex™ LH20 (Pharmacia) oder Biogel™ P2 100-200 mesh (BioRad). Es ist auch möglich, zuerst die Reaktionslösung zu gefriertrocknen und anschließend den festen Rückstand mit Methanol zu extrahieren, wobei nach Filtration und Einengen der methanolischen Lösung die obengenannten Chromatographiemethoden zur weiteren Reinigung eingesetzt werden können.

Die im folgenden aufgeführten Beispiele dienen zur weiteren Erläuterung der Erfindung.

**Beispiel 1**

Zu 1,24 g (4,1 mmol) β-ortho-Nitrophenylgalactosid und 2,0 g (7,5 mmol) (L)-Z-Serinethylester in 100 ml Kaliumphosphatpuffer (0,1 M, pH = 7; 10mM $MgCl_2$) gibt man 75 U β-Galactosidase aus E. coli (50 µl $(NH_4)_2SO_4$-Suspension, Boehringer Mannheim) und rührt 5 h bei Raumtemperatur.

Nach Gefriertrocknung, Extraktion des Rückstands mit Methanol, anschließender Flash-Chromatographie an Kieselgel ($MeOH/CH_2Cl_2$/Hexan = 1:6:6) und abschließender Reinigung durch präparative Dünnschichtchromatographie ($MeOH/CHCl_3$/Hexan = 2:6:6) erhält man 300 mg (17 %) des gewünschten Glycokonjugats, das nach $^1H$-/$^{13}C$-NMR als eine saubere Verbindung anfällt:
$^{13}C$-NMR ($DMSO-d_6$, 75 MHz, $\delta$ in ppm):
C1 (Gal): 104,03 ($\beta$)
FAB-MS: $MH^+$ = 430

**Beispiel 2:**

Zu 2,5 g (8,3 mmol) $\beta$-ortho-Nitrophenyl-Galactosid und 8,0 g (28,7 mmol) (L)-Z-Serinallylester in 200 ml Kaliumphosphatpuffer (0,07M, pH = 7; 10mM $MgCl_2$) gibt man 150 U $\beta$-Galactosidase aus E. coli (100 $\mu$l $(NH_4)_2SO_4$-Suspension, Boehringer Mannheim) und rührt 24 h bei Raumtemperatur. Aufarbeitung durch Gefriertrocknung, Extraktion des Rückstands mit Methanol und Säulenchromatographie an Kieselgel ($MeOH/CH_2Cl_2$/Hexan = 1:6:2) ergibt 435 mg (12 %) des gewünschten Glycokonjugats.
$^{13}C$-NMR ($DMSO-d_6$, 75 MHz, $\delta$ in ppm):
C1 (Gal): 104,06 ($\beta$)
FAB-MS: $MH^+$ = 442

**Beispiel 3:**

124 mg (0,41 mmol) $\beta$-ortho-Nitrophenylgalactosid und 200 mg (0,72 mmol) (L)-Z-Serinallylester werden mit 7,5 U $\beta$-Galactosidase aus E. coli (5 $\mu$l $(NH_4)_2SO_4$-Suspension, Boehringer Mannheim) in 10 ml Kaliumphosphatpuffer (0,07 M, pH = 7,0; 10 mM $MgCl_2$) 2 1/4 h bei 50 °C gerührt. Gefriertrocknung und präparative Dünnschichtchromatographie ($MeOH/CHCl_3$/Hexan = 2:2:6) ergeben 22 mg (12 %) des erwarteten Glycosids.

Die spektroskopischen Daten entsprechen Beispiel 2.

**Beispiel 4:**

225 mg (0,85 mmol) $\beta$-ortho-Nitrophenylgalactosid und 500 mg (1,8 mmol) (L)-Z-Serinallylester werden mit 15 U $\beta$-Galactosidase aus E. coli (10 $\mu$l $(NH_4)_2SO_4$-Suspension, Boehringer Mannheim), in 20 ml Kaliumphosphatpuffer (0,07 M, pH = 7,0; 10 mM $MgCl_2$) 2 1/4 h bei Raumtemperatur gerührt.
Nach Gefriertrocknung und Extraktion des Rückstands mit Methanol wird über Sephadex™ LH20 (Säule 60 x 2,5 cm) mit Methanol chromatographiert. Man erhält 34 mg (9 %) des gewünschten Produkts.

Die spektroskopischen Daten entsprechen Beispiel 2.

**Beispiel 5:**

Zu 538 mg (1,78 mmol) $\beta$-ortho-Nitrophenylgalactosid und 1,07 g (3,84 mmol) (D)-Z-Serinallylester in 50 ml TRIS-Puffer (0,1 M, pH = 7,2) gibt man 75 U $\beta$-Galactosidase aus E. coli (50 $\mu$l $(NH_4)_2SO_4$-Suspension, Boehringer Mannheim) und rührt 6,5 h bei Raumtemperatur. Nach Gefriertrocknung erfolgt Chromatographie über Biogel™ P2 (100-200 mesh, Säule 50 x 3 cm) mit Wasser und an Kieselgel mit $MeOH/CHCl_3$/Hexan 1:3:1 . Man erhält das gewünschte Glycokonjugat mit einer Ausbeute von 95 mg (12 %).
$^{13}C$-NMR ($DMSO-d_6$, 75 MHz, $\delta$ in ppm)
FAB-MS : $MH^+$ = 442

**Beispiel 6:**

Zu 542 mg (1,8 mmol) $\beta$-ortho-Nitrophenylgalactosid und 1,06 g (3,8 mmol) (L)-Z-Serinethylester in 50 ml TRIS-Puffer (0,01 M, pH = 7,2) gibt man 75 U $\beta$-Galactosidase aus E. coli (50 $\mu$l $(NH_4)_2SO_4$-Suspension, Boehringer Mannheim) und rührt 5,5 h bei Raumtemperatur.
Nachreinigung erfolgt durch Gefriertrocknung, Chromatographie mit Wasser an Biogel™ P2 (100-200 mesh, Säule 50 x 3 cm) und mit $CHCl_3$/MeOH/Hexan (3:1:1) an Kieselgel. Man erhält 78 mg des gewünschten Glycosids (10 % Ausbeute).

Die spektroskopischen Daten entsprechen Beispiel 1.

**Beispiel 7:**

532 mg (1,77 mmol) β-ortho-Nitrophenylgalactosid und 1,02 g (4,16 mmol) (L)-Boc-Serinallylester werden mit 75 U β-Galactosidase aus E. coli (50 µl $(NH_4)_2SO_4$-Suspension, Boehringer Mannheim) in 50 ml TRIS-Puffer (0,01 M, pH = 7,2) 5,5 h bei Raumtemperatur gerührt. Gefriertrocknung, Chromatographie mit Wasser an Biogel P2 (100-200 mesh, Säule 50 x 3 cm) und mit $CHCl_3$/MeOH/Hexan (3:1:1) an Kieselgel ergeben 130 mg (18 %) des gewünschten Glycokonjugats.

Die Verbindung ist nach DC, $^1H/^{13}C$-NMR und FAB-MS einheitlich.
$^{13}C$-NMR (DMSO-$d_6$, 75 MHz, δ in ppm):
C1 (Gal): 104,01 (β)
FAB-MS: $MH^+$ = 408.

**Beispiel 8:**

391 mg (1,3 mmol) α-p-Nitrophenylmannosid und 714 mg (2,9 mmol) (L)-Z-Serinallylester werden mit 22 U (220 µl $(NH_4)_2SO_4$-Suspension) α-Mannosidase (aus Jack Beans, Sigma) in 35 ml Tris-Puffer (pH 6, 0,01 M) 3,5 h bei Raumtemperatur gerührt.
Nach Gefriertrocknung und Chromatographie an 90 g Emosil-Bioselect™ (100-30 C18 in Acetonitril-Wasser 40:60)und Kieselgel ($CHCl_3$/Methanol/Hexan 3:1:1), erhält man 22 g (4 %) des gewünschten Produkts, das nach $^1H/^{13}C$-NMR und FAB-MS einheitlich ist.
$^{13}C$-NMR (DMSO-$d_6$, 75 MHz, δ in ppm):
C1 (Man): 100,61 (α)
FAB-MS: $MH^+$ = 442.

| Beispiel | Verbindung der Formel II | Verbindung der Formel III $R^2$ | $R^3$ (*) | Enzym Art, | [units] | Menge [mmol] II | III | Puffer (**) [ml] | Dauer/Temp. | Ausbeute [%] | Aufarbeitung analog Beispiel |
|---|---|---|---|---|---|---|---|---|---|---|---|
| 9 | α-F-Glc | Z | OAllyl | A, | 8,4 | 0,7 | 0,80 | 10 | 7 h, RT | 5 | 1 |
| 10 | ($R^1$ = F) | Z | OAllyl | B | | 2,5 | 0,75 | 10 | 21 h, RT | 2 | 1 |
| 11 | | Z | OAllyl | C, | 500 | 2,5 | 0,75 | 10 | 21 h, RT | 2 | 1 |
| 12 | ortho-Nitro-phenyl-β-Galactosid | Ac | OHeptyl | C, | 500 | 2,5 | 0,75 | 10 | 21 h, RT | 12 | 8 |
| 13 | | Z | OAllyl | D, | 15 | 0,42 | 1,54 | 10 | 3 h, RT | 9 | 5 |
| 14 | | Z | OAllyl | D, | 15 | 0,42 | 0,72 | 10 | 2 h, RT | 12 | 5 |
| 15 | | Z | OAllyl | D, | 1,5 | 0,42 | 1,4 | 10 | 25 h, RT | 6 | 5 |
| 16 | | Z | OAllyl | D, | 7,5 | 0,42 | 0,72 | 10 | 2,5 h, RT | 8 | 5 |
| 17 | | Z | OAllyl | D, | 7,5 | 0,42 | 0,72 | 5 + 5 ml Toluol | 4 ¾ h, RT | 6 | 5 |
| 18 | | Z | OAllyl | D, | 7,5 | 0,42 | 0,72 | 5 + 5 ml Xylol | 4 ¾ h, RT | 8 | 5 |
| 19 | | Ac | OAllyl | D, | 75 | 4,2 | 1,8 | 50 ml (***) | 5,5 h, RT | 19 | 5 |
| 20 | | Z | OMe | D, | 225 | 4,2 | 16,0 | 100 | 6 h, RT | 10 | 5 |

* Verbindung der L-Reihe
** Wenn nicht anders angegeben: Kaliumphosphatpuffer (0,07 M; pH = 7; 10 mM $MgCl_2$)
*** TRIS-Puffer (0,1 M, pH = 7,2)

| Beispiel | Verbindung der Formel II | Verbindung der Formel III $R^2$ | $R^3$ (*) | Enzym Art, | [units] | Menge [mmol] II | III | Puffer (**) [ml] | Dauer/Temp. | Ausbeute [%] | Aufarbeitung analog Beispiel |
|---|---|---|---|---|---|---|---|---|---|---|---|
| 21 | | Aloc | OBn | D, | 7,5 | 0,4 | 0,8 | 10 | 21 h, RT | 5 | 8 |
| 22 | | Aloc | OEtBr | D, | 7,5 | 0,4 | 0,8 | 10 | 6 h, RT | 2 | 1 |
| 23 | | Z | LeuOMe | D, | 7,5 | 0,4 | 0,7 | 10 | 7 h, RT | 5 | 3 |
| 24 | | Aloc | PheOMe | D, | 7,5 | 0,4 | 0,7 | 10 | 6 h, RT | 2 | 3 |
| 25 | p-Nitrophenyl-β-Mannosid | Z | OAllyl | E, | 0,5 | 0,03 | 0,09 | 2 | 170 h, RT | 2 | 3 |

A: α-Glucosidase (Maltase) aus Hefe, EC 3.2.1.20 von Boehringer Mannheim

B: Transglucosidase von Amano Pharmaceutical Co.

C: α-Amylase aus Aspergillus oryzae, EC 3.2.1.1, von Sigma Chemie GmbH

D: β-Galactosidase aus E. coli EC 3.2.1.23 von Boehringer Mannheim

E: β-Mannosidase, snail acetone powder, EC 3.2.1.25 von Sigma Chemie GmbH

* Verbindungen der L-Reihe

** Wenn nicht anders angegeben: Kaliumphosphatpuffer (0,07 M; pH = 7; 10 mM $MgCl_2$

## Patentansprüche

**1.** Verfahren zur Herstellung von Glycokonjugaten der Formel I,

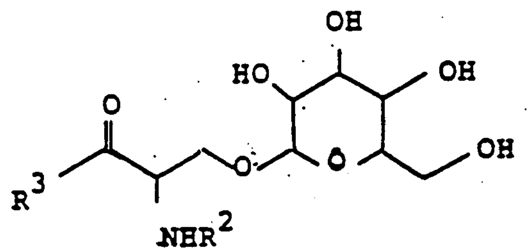

I

in der $R^2$ eine Aminoschutzgruppe und $R^3$ eine Hydroxylgruppe sowie eine Alkoxy- oder Thioalkylgruppe oder eine Alkenyloxygruppe jeweils mit 1 bis 18 C-Atomen, die mit Halogen oder Cyan substituiert sein können, eine Aryloxygruppe mit 6 bis 10 C-Atomen, die mit Alkyl-, Alkoxy-, Thioalkyl-, jeweils mit 1 bis 5 C-Atomen, und Nitrogruppen substituiert sein kann oder die Gruppe -$NHR^4$ ist, in der $R^4$ eine Alkylgruppe mit 1 bis 5 C-Atomen oder ein Rest der Formel IV

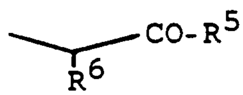

IV

oder ein Di- oder Tripeptid-Rest der Formel V oder VI

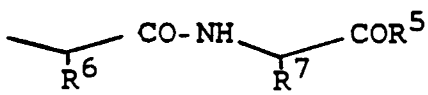

V

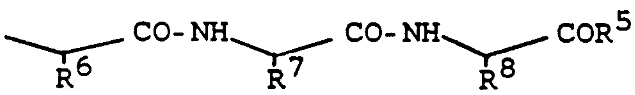

VI

ist, wobei
$R^5$ eine Hydroxylgruppe, eine Alkoxy-, eine Thioalkyl oder eine Alkenyloxygruppe, mit jeweils 1 bis 5 C-Atomen, die mit Halogen oder Cyan substituiert sein kann, eine Aryloxygruppe mit 6 bis 10 C-Atomen, die mit Alkyl-, Alkoxy-, Thioalkyl-, mit jeweils 1 bis 5 C-Atomen, und Nitrogruppen substituiert sein kann und
$R^6$, $R^7$, $R^8$, die gleich oder verschieden sind, Wasserstoff oder geradkettige, verzweigte oder cyclische Alkyl- oder Alkenylgruppen mit 1 bis 10 C-Atomen, die mit Halogen, Hydroxyl, Alkoxy, Thiol, Thioalkyl, Aryl oder Heteroaryl substituiert sein können, darstellen,
dadurch gekennzeichnet, daß die Verbindung der Formel II,

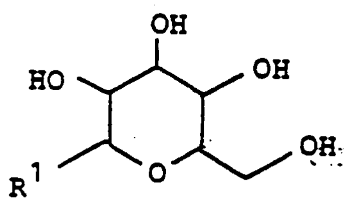

II

in der $R^1$ Fluor, eine Hydroxylgruppe, eine Alkoxygruppe mit 1 bis 5 C-Atomen, eine Alkenyloxygruppe mit 2 bis 5 C-Atomen, eine Aryloxygruppe mit 6 bis 10 C-Atomen oder ein über ein Sauerstoffatom gebundener Kohlenhydratrest ist, mit der Verbindung der Formel III,

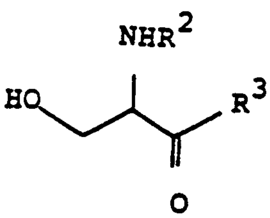

III

in Gegenwart einer Glycosidase inkubiert wird.

**2.** Verfahren nach Anspruch 1, dadurch gekennzeichnet, daß die Verbindung der Formel II, in der $R^1$ Fluor, eine Methoxygruppe, eine Allyloxygruppe, eine Vinyloxygruppe, eine Phenoxygruppe, eine ortho- oder para-Nitrophenoxygruppe oder eine Dinitrophenoxygruppe bedeutet, eingesetzt wird.

**3.** Verfahren nach Anspruch 1, dadurch gekennzeichnet, daß die Verbindung der Formel III, in der $R^2$ Benzyloxycarbonyl, Allyloxycarbonyl, tertiär-Butyloxycarbonyl, Formyl, Acetyl, Chloracetyl, Trifluoracetyl, Phenacetyl, Benzoyl oder einen Acylrest einer längerkettigen Fettsäure mit 6 bis 24 C-Atomen bedeutet, eingesetzt wird.

**4.** Verfahren nach Anspruch 1 oder 3, dadurch gekennzeichnet, daß die Verbindung der Formel III, in der $R^3$ Methoxy, Methoxymethyl, Benzyloxymethyl, Methylthiomethyl, Ethoxy, Chlorethoxy, Bromethoxy oder Cyanethoxy, Benzyloxy, p-Nitrobenzyloxy, p-Methoxybenzyloxy, Piperonyloxy, Allyloxy oder Vinyloxy sowie tertiär-Butyloxy oder tertiär-Butyl dimethylsilyloxy ist oder, wenn $R^3$ die Gruppe $NHR^4$ bedeutet, $R^4$ die Gruppe der Formel IV oder V ist in der $R^5$ die Bedeutung wie für $R^3$ definiert hat, wobei $R^3$ und $R^5$ gleich oder verschieden sein können, eingesetzt wird.

**5.** Verfahren nach einem oder mehreren der Ansprüche 1 bis 4, dadurch gekennzeichnet, daß die Verbindungen der Formel II und der Formel III im Verhältnis 4 zu 1 bis 1 zu 10 eingesetzt werden.

**6.** Verfahren nach einem oder mehreren der Ansprüche 1 bis 5, dadurch gekennzeichnet, daß die Reaktion in einem pH-Bereich von 5,0 bis 8,0 abläuft.

**7.** Verfahren nach einem oder mehreren der Ansprüche 1 bis 6, dadurch gekennzeichnet, daß die Reaktion in einem Temperaturbereich von -30 bis 50 °C abläuft.

**8.** Verfahren nach einem oder mehreren der Ansprüche 1 bis 7, dadurch gekennzeichnet, daß die Glucosidase aus Hefe und Süßmandeln, Galactosidase aus E. coli, Aspergillus niger, Kaffeebohnen oder Rindertestes, $\beta$-Mannosidase in Form von snail acetone powder bzw. $\alpha$-Mannosidase aus Jackbeans oder Mandeln, Amylase aus Aspergillus oryzae, Bacillus subtilis oder Schweinepankreas oder Amyloglucosidasen aus Aspergillus niger als Glucosidase eingesetzt werden.

## Claims

1. A process for preparing glyco conjugates of the formula I

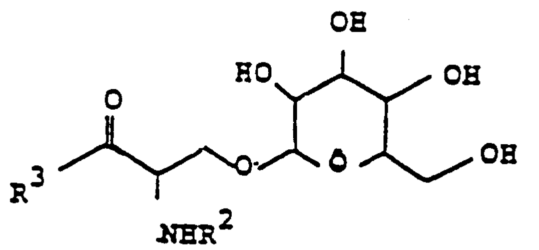

I

in which $R^2$ is an amino protective group and $R^3$ is a hydroxyl group and an alkoxy or alkylmercapto group or an alkenyloxy group, each of which has 1 to 18 carbon atoms and can be substituted by halogen or cyano, or an aryloxy group which has 6 to 10 carbon atoms and can be substituted by alkyl, alkoxy, alkylmercapto, in each case with 1 to 5 carbon atoms, and nitro groups, or the group $-NHR^4$ in which $R^4$ is an alkyl group with 1 to 5 carbon atoms or a radical of the formula IV

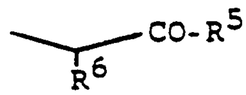

IV

or a di- or tripeptide residue of the formula V or VI

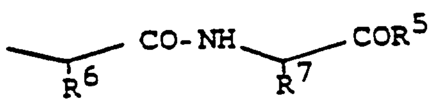

V

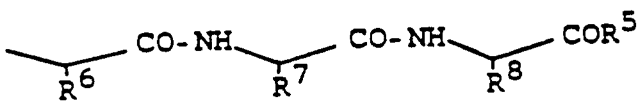

VI

where $R^5$ is a hydroxyl group, an alkoxy, an alkylmercapto or an alkenyloxy group, each of which has 1 to 5 carbon atoms and can be substituted by halogen or cyano, or an aryloxy group which has 6 to 10 carbon atoms and can be substituted by alkyl, alkoxy, alkylmercapto, each of which has 1 to 5 carbon atoms, and nitro groups, and $R^6$, $R^7$ and $R^8$, which are identical or different, are hydrogen or straight-chain, branched or cyclic alkyl or alkenyl groups which have 1 to 10 carbon atoms and can be substituted by halogen, hydroxyl, alkoxy, mercapto, alkylmercapto, aryl or heteroaryl,
which comprises incubating the compound of the formula II

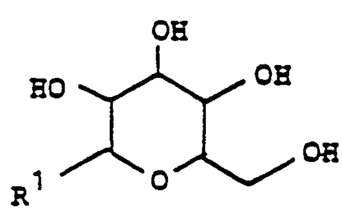

II

in which $R^1$ is fluorine, a hydroxyl group, an alkoxy group with 1 to 5 carbon atoms, an alkenyloxy group with 2 to 5 carbon atoms, an aryloxy group with 6 to 10 carbon atoms or a carbohydrate residue which is bonded via an oxygen atom, with the compound of the formula III

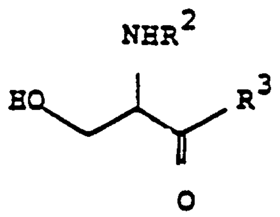

III

in the presence of a glycosidase.

2. The process as claimed in claim 1, wherein the compound of the formula II in which $R^1$ is fluorine, a methoxy group, an allyloxy group, a vinyloxy group, a phenoxy group, an ortho- or para-nitrophenoxy group or a dinitrophenoxy group is employed.

3. The process as claimed in claim 1, wherein the compound of the formula III in which $R^2$ is benzyloxycarbonyl, allyloxycarbonyl, tertiarybutyloxycarbonyl, formyl, acetyl, chloroacetyl, trifluoroacetyl, phenacetyl, benzoyl or an acyl radical of a long-chain fatty acid with 6 to 24 carbon atoms is employed.

4. The process as claimed in claim 1 or 3, wherein the compound of the formula III in which $R^3$ is methoxy, methoxymethyl, benzyloxymethyl, methylmercaptomethyl, ethoxy, chloroethoxy, bromoethoxy or cyanoethoxy, benzyloxy, p-nitrobenzyloxy, p-methoxybenzyloxy, piperonyloxy, allyloxy or vinyloxy and tertiary-butyloxy or tertiary-butyldimethylsilyloxy or, when $R^3$ is the group $NHR^4$, $R^4$ is the group of the formula IV or V in which $R^5$ has the meaning as defined for $R^3$, it being possible for $R^3$ and $R^5$ to be identical or different, is employed.

5. The process as claimed in one or more of claims 1 to 4, wherein the compounds of the formula II and of the formula III are employed in the ratio 4:1 to 1:10.

6. The process as claimed in one or more of claims 1 to 5, wherein the reaction takes place in a pH range from 5.0 to 8.0.

7. The process as claimed in one or more of claims 1 to 6, wherein the reaction takes place in a temperature range from -30 to 50 °C.

8. The process as claimed in one or more of claims 1 to 7, wherein the glucosidase from yeast and sweet almonds, galactosidase from E. coli, Aspergillus niger, coffee beans or bovine testes, $\beta$-mannosidase in the form of snail acetone powder or $\alpha$-mannosidase from jackbeans or almonds, amylase from Aspergillus oryzae, Bacillus subtilis or pig pancreas or amyloglucosidases from Aspergillus niger are employed as glucosidase.

## Revendications

**1.** Procédé pour la préparation de glycoconjugués de formule 1

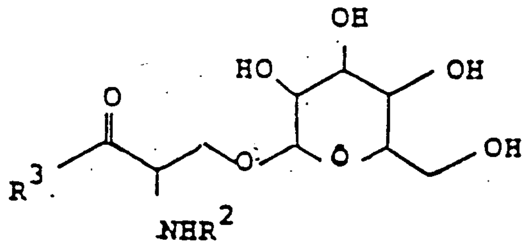

I

dans laquelle $R^2$ représente un groupe protecteur de fonction amino et $R^3$ représente un groupe hydroxy ou un radical alcoxy, thioalkyle ou alcényloxy ayant chacun de 1 à 18 atomes de carbone, qui peuvent être substitués par des atomes d'halogène ou par le groupe cyano, un radical aryloxy ayant de 6 à 10 atomes de carbone, qui peut être substitué par des groupes alkyle, alcoxy, thioalkyle ayant chacun de 1 à 5 atomes de carbone ou par le groupe nitro, ou est le radical $-NHR^4$, dans lequel $R^4$ représente un groupe alkyle ayant de 1 à 5 atomes de carbone, ou est un radical de formule IV

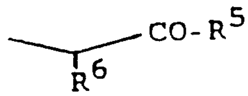

IV

ou un reste de di- ou tripeptide de formule V ou VI

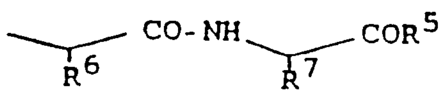

V

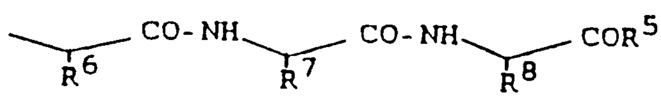

VI

$R^5$ étant un groupe hydroxy ou un groupe alcoxy, thioalkyle ou alcényloxy ayant chacun de 1 à 5 atomes de carbone, qui peuvent être substitués par des atomes d'halogène ou par le groupe cyano, un radical aryloxy ayant de 6 à 10 atomes de carbone, qui peut être substitué par des groupes alkyle, alcoxy, thioalkyle ayant chacun de 1 à 5 atomes de carbone ou par le groupe nitro, et
$R^6$, $R^7$, $R^8$, qui peuvent être identiques ou différents, représentant un atome d'hydrogène ou des groupes alkyle ou alcényle à chaîne droite, ramifiés ou cycliques ayant de 1 à 10 atomes de carbone, qui peuvent être substitués par des atomes d'halogène ou par des groupes hydroxy, alcoxy, thiol, thioalkyle, aryle ou hétéroaryle,
caractérisé en ce que l'on met à incuber un composé de formule II

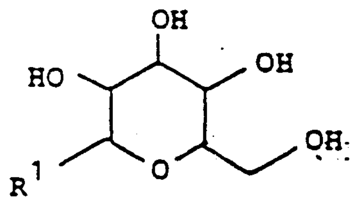

II

dans laquelle $R^1$ est un atome de fluor ou un groupe hydroxy, alcoxy ayant de 1 à 5 atomes de carbone, alcényloxy ayant de 2 à 5 atomes de carbone, aryloxy ayant de 6 à 10 atomes de carbone, ou un reste glucidique lié par un atome d'oxygène, avec un composé de formule III

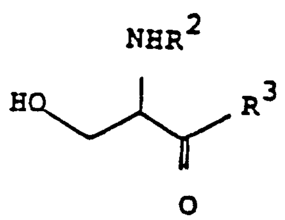

III

en présence d'une glucosidase.

**2.** Procédé selon la revendication 1, caractérisé en ce que l'on utilise un composé de formule II dans lequel $R^1$ représente un atome de fluor ou un groupe méthoxy, allyloxy, vinyloxy, phénoxy, o- ou p-nitrophénoxy, ou dinitrophénoxy.

**3.** Procédé selon la revendication 1, caractérisé en ce que l'on utilise un composé de formule III dans lequel $R^2$ représente le groupe benzyloxycarbonyle, allyloxycarbonyle, tert-butyloxycarbonyle, formyle, acétyle, chloracétyle, trifluoroacétyle, phénacétyle, benzoyle ou un reste acyle d'un acide gras à longue chaîne ayant de 6 à 24 atomes de carbone.

**4.** Procédé selon la revendication 1 ou 3, caractérisé en ce que l'on utilise un composé de formule III dans lequel $R^3$ représente le groupe méthoxy, méthoxyméthyle, benzyloxyméthyle, méthylthiométhyle, éthoxy, chloréthoxy, bromoéthoxy ou cyanoéthoxy, benzyloxy, p-nitrobenzyloxy, p-méthoxybenzyloxy, pipéronyloxy, allyloxy ou vinyloxy, ou tert-butyloxy ou tert-butyldiméthylsilyloxy, ou bien, lorsque $R^3$ représente le radical $NHR^4$, $R^4$ est un groupe de formule IV ou V dans lequel $R^5$ a la signification donnée pour $R^3$, $R^3$ et $R^5$ pouvant être identiques ou différents.

**5.** Procédé selon une ou plusieurs des revendications 1 à 4, caractérisé en ce que l'on utilise les composés de formule II et de formule III en un rapport allant de 4:1 à 1:10.

**6.** Procédé selon une ou plusieurs des revendications 1 à 5, caractérisé en ce que la réaction se déroule dans un intervalle de pH allant de 5,0 à 8,0.

**7.** Procédé selon une ou plusieurs des revendications 1 à 6, caractérisé en ce que la réaction se déroule dans une plage de températures allant de -30 à 50 °C.

**8.** Procédé selon une ou plusieurs des revendications 1 à 7, caractérisé en ce que, en tant que glucosidase, on utilise la glucosidase de levure et d'amandes douces, la galactosidase de *E. coli, d'Aspergillus niger*, de grains de café ou de testicules bovins, la $\beta$-mannosidase sous forme de poudre acétinique d'escargot ou l'$\alpha$-mannosidase de pois Canavalia *ensiformis* ou d'amandes, l'amylase *d'Aspergillus oryzae*, de *Bacillus subtilis* ou de pancréas porcin, ou des amyloglucosidases *d'Aspergillus niger*.